# FASCICULE DE BREVET EUROPEEN

(11) **EP 2 393 441 B1**
(45) Date de publication et mention de la délivrance du brevet: **12.08.2015**
(21) Numéro de dépôt: 10707077.3
(22) Date de dépôt: 09.02.2010
(51) Int. Cl.: A61B 17/86

(54) **VIS D'OSTEOSYNTHESE ET D'ARTHRODESE**
OSTEOSYNTHESE- UND ARTHRODESE-SCHRAUBE
SCREW FOR OSTEOSYNTHESIS AND ARTHRODESIS

(30) Priorité: 09.02.2009 FR 0900557
(43) Date de publication de la demande: 14.12.2011
(73) Titulaire: Memometal Technologies, 35170 Bruz (FR)
(72) Inventeur: PRANDI, Bernard, F-35700 Rennes (FR); LARIVIERE, Jean, F-59700 Marcq en Baroueul (FR); R'TAIMATE, Mohammed, F-59273 Peronne en Melantois (FR)
(74) Mandataire: Benech, Frédéric
(86) Numéro de dépôt international: PCT/FR2010/000094
(87) Numéro de publication internationale: WO 2010/089481

(56) Documents cités:
- EP-A- 0 856 293
- EP-A- 1 378 205
- WO-A-2004/069031
- US-A1- 2004 073 218
- US-B1- 6 306 140

## Description

L'invention concerne une vis d'ostéosynthèse et d'arthrodèse pour la chirurgie orthopédique.

Elle trouve une application particulièrement importante, bien que non exclusive dans le domaine des implants pour la chirurgie orthopédique de la main et du pied plus particulièrement au niveau des phalanges des doigts et des orteils, mais également des métacarpiens et métatarsiens, et ce notamment pour la chirurgie correctrice de la main et du pied.

On rappelle qu'un implant d'ostéosynthèse doit servir à maintenir en place deux parties (ou plus) d'un même os fracturé ou coupé par une opération de chirurgie (ostéotomie), et ce le temps nécessaire à la consolidation de cet os (typiquement trois mois).

Une arthrodèse est quant à elle le blocage d'une articulation par voie chirurgicale pour souder deux os en un seul, au moyen d'un dispositif d'ostéosynthèse.

De façon générale le but de toute ostéosynthèse, et particulièrement dans le cas d'une arthrodèse, est de rechercher une très bonne stabilité de l'implant afin d'obtenir la consolidation dans les meilleures conditions, c'est-à-dire dans la position choisie par le chirurgien, en minimisant les problèmes de douleur post-opératoire et les oedèmes, et en raccourcissant autant que faire se peut le temps de consolidation.

Pour obtenir ce résultat, la forme de l'implant est critique.

Un autre but recherché est d'apporter et maintenir une légère compression entre les parties d'os à fusionner, ce qui facilite la consolidation. Ici encore la forme de l'implant est importante.

Différentes solutions techniques ont déjà été proposées, pour réaliser une telle ostéosynthèse, notamment au niveau des extrémités (pied, main, poignet, cheville, ...).

Outre des agrafes classiques ou à mémoire de forme, il est par exemple courant d'utiliser des vis, notamment des vis à double pas permettant la mise en compression. Dans ces vis, le pas de chaque extrémité est différent (pas distal et pas proximal ou de tête), par exemple de 0,25 mm par tour, ce qui permet d'obtenir une compression de 1 mm en 4 tours. Ces vis connues peuvent être canulées (montage sur une broche) ou non. Elles sont en général auto-taraudantes, c'est-à-dire qu'elles ne nécessitent pas en général de perçage.

Le document US 6 306 140 B1 décrit un vis d'ostéosynthèse selon le préambule de la revendication 1.

Le document FR 2 840 799 décrit une vis auto-forante en partie distale, pour laquelle il est néanmoins souvent nécessaire de réaliser un vrai préperçage notamment en partie proximale ce qui enlève l'intérêt de l'auto-forant. Par ailleurs les arêtes de coupe des filets sont définies au niveau de l'alésage, ce qui fragilise la vis, c'est-à-dire qu'en pratique les dents ainsi définies cassent très facilement.

Un deuxième problème fréquent de ces vis est que leur longueur doit être parfaitement adaptée au site osseux pour ne pas créer de gêne, et donc qu'elles doivent se visser immédiatement au contact de l'os sans repousser celui-ci au niveau distal, tout en assurant un bon ancrage de l'extrémité ce qui implique une extrémité cylindrique antinomique d'une bonne pénétration.

On connaît ainsi (US 6,306,140) une vis comprenant trois parties, à savoir une partie distale taraudée cylindrique, une partie centrale lisse et une partie proximale ou de tête taraudée autoperforante de plus gros diamètre.

Une telle vis ne présente une condition angulaire particulière que sur la tête, la partie distale étant donc et quant à elle classique, c'est à dire avec les arrêtes du pas de vis s'inscrivant dans un cylindre.

Le document EP 0 856 293 A1 décrit quant à lui une vis cette fois ci auto-taraudeuse et auto-forante mais qui pour se faire présente des chanfreins aux extrémités ce qui retire par contre de la tenue à la vis ou oblige à l'enfouir plus profondément afin que l'os soit au niveau où la vis est totalement cylindrique. Par ailleurs les arêtes coupantes proposées sont trop profondes, fragiles et donc cassantes au niveau du fût.

L'invention remédie à tous ces inconvénients d'une manière simple, fiable, efficace et rationnelle.

Pour ce faire la présente invention a pour but de proposer une vis répondant mieux que celles antérieurement connues aux exigences de la pratique notamment en ce qu'elle propose une vis auto-forante et auto-taraudeuse, compressive, permettant une bonne pénétration et assurant simultanément un très bon ancrage osseux, et ce sans dépasser de l'os.

Une telle vis est spécialement adaptée aux techniques chirurgicales qui prennent appui des deux cotés sur une corticale assez fine comme dans le cas d'une ostéotomie métatarsienne de type « scarf ».

Dans la suite du texte, on se référera aux définitions suivantes :
- extrémité distale de la vis : correspond à la partie qui entre en contact en premier avec l'os ;
- extrémité proximale (ou tête) : correspond à la partie qui se visse en dernier et qui comprend la connexion à un moyen de vissage (tournevis par exemple) ;
- fût de la vis : correspond à l'âme ou partie globalement cylindrique sur laquelle est positionnée le pas de vis.

Dans ce but, l'invention propose donc, et notamment, une vis d'ostéosynthèse et d'arthrodèse pour la chirurgie orthopédique notamment de la main et du pied, canulée ou non, présentant le long de son axe principal (AP) trois parties successives, à savoir une partie distale comprenant des filets (A1), une partie centrale ou fût central lisse (f) et une partie proximale ou tête comprenant des filets (A2), la partie distale ayant un diamètre externe légèrement inférieur à celui de la partie proximale et présentant un pas de vis légèrement supérieur à celui de la partie proximale, permettant une mise en compression des deux parties osseuses à souder, la zone d'attaque de l'os en partie proximale (A2a) étant conique auto-perforante, caractérisé en ce que l'extrémité de la partie distale est conique,
en ce que la somme des angles a1+b1 en distal, a2+b2 en proximal, définis entre, d'une part, l'axe principal de la vis (AP) et la conicité extérieure du fût ((f1) en distal et (f2) en proximal, à savoir (a1) en distal, et (a2) en proximal et, d'autre part, entre l'axe principal de la vis (AP) et la ligne de crête des filets (P) du pas de vis, à savoir (b1) en distal, et (b2) en proximal, est supérieur à 45,
et en ce que la partie d'attaque c'est à dire la plus distale de chaque filet présente une pluralité d'arêtes coupantes (AR) obtenues par enlèvement de matière.

Par diamètre légèrement inférieur on entend une différence comprise entre 1/5 et 1/20^{e} du diamètre de la partie proximale et avantageusement et par exemple un diamètre de la partie distale égal à de l'ordre de 9/10^{e} dudit diamètre de la partie proximale.

Par pas de vis légèrement supérieur on entend un pas de vis de la partie distale supérieur d'une valeur comprise entre 5% et 20%, et avantageusement de l'ordre de 10% du pas de vis de la partie proximale.

Les deux zones filetées, qu'elles soient distales ou proximales, permettent quant à elles de réaliser l'ancrage osseux, chacun des deux filets étant auto-forant et auto-taraudant.

L'invention part de l'idée de réaliser en combinaison deux zones d'ancrage mordant dans l'os au vissage, l'une pour la partie proximale et l'autre pour la partie distale, avec des profils extérieurs particuliers, à savoir légèrement coniques (plus fin coté distal) sur lesquels sont taillés les filetages également coniques, toutes deux, en distale et en proximale, comprenant plusieurs arêtes coupantes réalisées chacune par une entaille longitudinale dans le pas de vis.

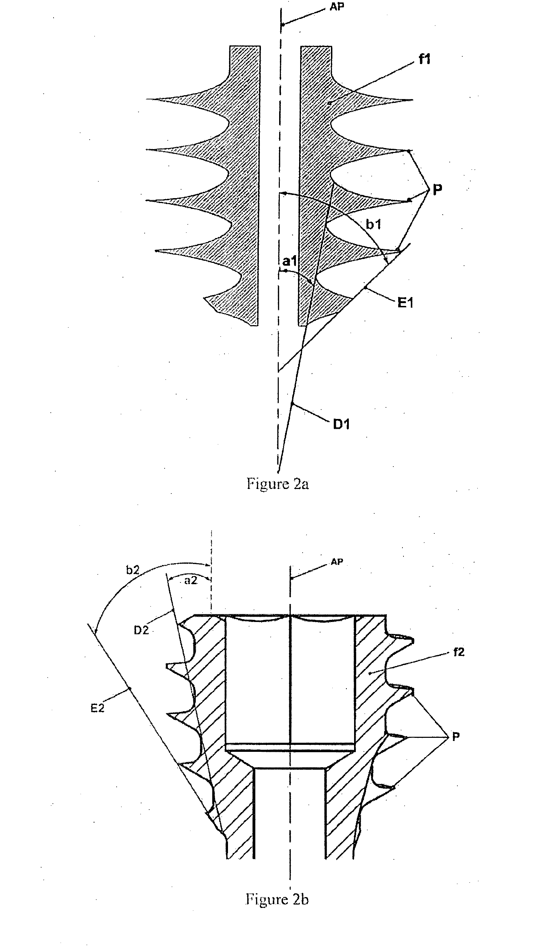

Plus précisément dans le mode de réalisation plus particulièrement décrit ici, la partie distale dont le corps est de conicité faible (angle a1 par exemple compris entre 4 et 15°) est munie d'un filetage de forte conicité (angle b1 par exemple compris entre 50 et 80°) de sorte que le profil de la crête des filets de l'extrémité de la partie distale devient cylindrique après le premier ou le deuxième filet (voir figure 2a).

Une telle disposition combinée avec des arêtes particulièrement coupantes de part leur formation par enlèvement de matière, permet à l'extrémité de jouer un rôle de pointeau qui devient immédiatement accrochant et qui permet de placer la vis sans préperçage dans un os mou ou avec un léger pointage dans l'os dans un os plus dur, tout en ayant la même capacité d'ancrage qu'une vis à profil de crête purement cylindrique.

On s'aperçoit par ailleurs que le fait que les arêtes soient obtenues par enlèvement de matière et le fait que les profils soient spécifiques et tels que décrits ci-avant, autorisent de façon surprenante, non seulement une bonne pénétration immédiate dans les deux parties de l'os, mais également une bonne compression des deux parties d'os l'une contre l'autre et enfin une bonne tenue dans l'os lui-même, et ce malgré la conicité de la partie distale de la vis, et sans dépasser de l'os.

L'ensemble est implantable par voie traditionnelle des vis, après stabilisation de l'ostéotomie par tout instrument adapté (pince ou davier) et à l'aide d'un tournevis adapté, montée sur une broche (vis canulée) ou non (vis non canulée), ou d'un embout de vissage permettant de se connecter à un moteur.

Par ailleurs pour tenir compte des caractéristiques anatomiques, les zones d'ancrages sont avantageusement reliées à la zone médiane servant de résistance (notamment au cisaillement) au niveau du foyer d'ostéosynthèse par des zones de liaisons plus ou moins longues.

Enfin, le matériau constitutif de l'implant objet de l'invention permet un forage dans l'os et présente donc la dureté nécessaire. N'importe quel matériau implantable suffisamment dur tel que de l'acier inoxydable, du titane, un alliage CrCo (chrome-cobalt), ... est ici avantageusement choisi.

Dans un mode particulier de l'invention, l'implant est ainsi et notamment en alliage de titane tel que l'alliage connu sous la dénomination TA6V (alliage titane, aluminium, vanadium).

Dans des modes de réalisation avantageux on a de plus recours à l'une et/ou à l'autre des dispositions suivantes :
- les arêtes coupantes (AR) sont décalées en négatif par rapport à un axe radial à la vis, c'est-à-dire en arrière par rapport au sens de rotation de la vis au vissage ;
- les arêtes coupantes (AR) sont décalées en arrière pour présenter un angle d'attaque de l'os au vissage (C) en distal et en proximal) compris entre 5° et 12° ;
- les arêtes coupantes (AR) sont préférentiellement au nombre de trois ;
- la profondeur des arêtes coupantes (AR) est inférieure ou égale à la profondeur du pas de vis ;
- la profondeur des arêtes coupantes (AR) est égale à la profondeur du pas de vis ;
- la somme des angles (a1+b1) en distal, respectivement (a2+b2 en proximal, est comprise entre 50° et 70° ;
- l'angle (a1) en distal est compris entre 4° et 10° et l'angle (a2) en proximal est compris entre 10° et 20° ;
- les arêtes coupantes (AR) présentent une dépouille de 10° environ en arrière dans le sens de vissage ;
- la vis présente une tête plate ou conique.

L'invention sera mieux comprise à la lecture de la description qui suit d'un mode de réalisation donné à titre d'exemple non limitatif.

La description se réfère aux dessins qui l'accompagne dans lesquels :
La figure 1 est une vue latérale d'une vis selon un premier mode de réalisation de l'invention,
Les figures 2a et 2b sont des vues en coupe longitudinale selon l'axe principal de la vis, respectivement de la partie distale et de la partie proximale.
Les figures 3a et 3b montrent des coupes transversales au niveau des arêtes coupantes dans une position de référence (figure 3a) et après un vissage faible (environ 1/30^{ème} de tour) (figure 3b).

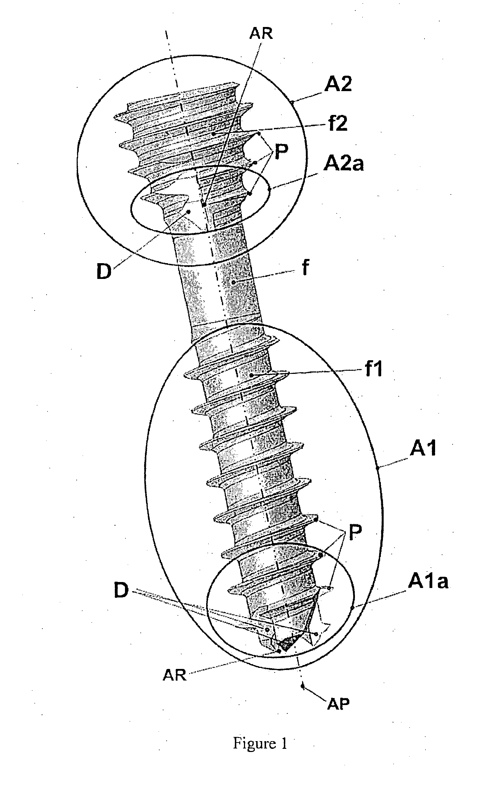

La figure 1 montre une vue générale de la vis selon le mode de réalisation de l'invention plus particulièrement décrit ici, qui présente deux zones d'ancrages, à savoir A1 en partie distale et A2 en partie proximale. L'âme, sur laquelle est positionnée le pas de vis, également appelée fût f, est globalement cylindrique. Pour la cohérence, f1 est la partie du fût de la zone A1 en distal, c'est à dire le corps de la vis abstraction faite des filets, et f2 est la partie du fût de la zone A2 en proximal. Chaque zone d'ancrage comporte des filets P et est composée d'une zone d'attaque (la première à venir en contact avec l'os et à mordre dedans au vissage) A1a en distal, et respectivement A2a en proximal suivi d'une zone de vissage.

Les zones d'attaque A1a en distal, et respectivement A2a en proximal comprennent chacune plusieurs dents D, par exemple trois dents définissant chacune une arête coupante AR. Ces dents sont réalisées par une saignée ou entaille parallèle à l'axe principal de la vis AP afin de créer une arête coupante AR, intersection de ce fraisage avec le diamètre extérieur du filet de la vis P.

La figure 2a montre plus précisément une coupe longitudinale de la vis en partie distale.

Sur cette coupe on définit a1 comme l'angle entre l'axe principal AP et la droite D1 reliant les fonds de filets P, ou correspondant à la surface extérieure du fût f1 abstraction faite des filets. On définit également l'angle b1 comme l'angle entre l'axe principal AP et la droite E1 reliant les crêtes des filets P.

De la même manière, la figure 2b montre une coupe longitudinale de la vis en partie proximale. Sur cette coupe on définit a2 comme l'angle entre l'axe principal AP et la droite D2 reliant les fonds de filets P, ou correspondant à la surface extérieure du fut f2 abstraction faite des filets.

On définit également l'angle b2 comme l'angle entre l'axe principal AP et la droite E2 reliant les crêtes des filets P.

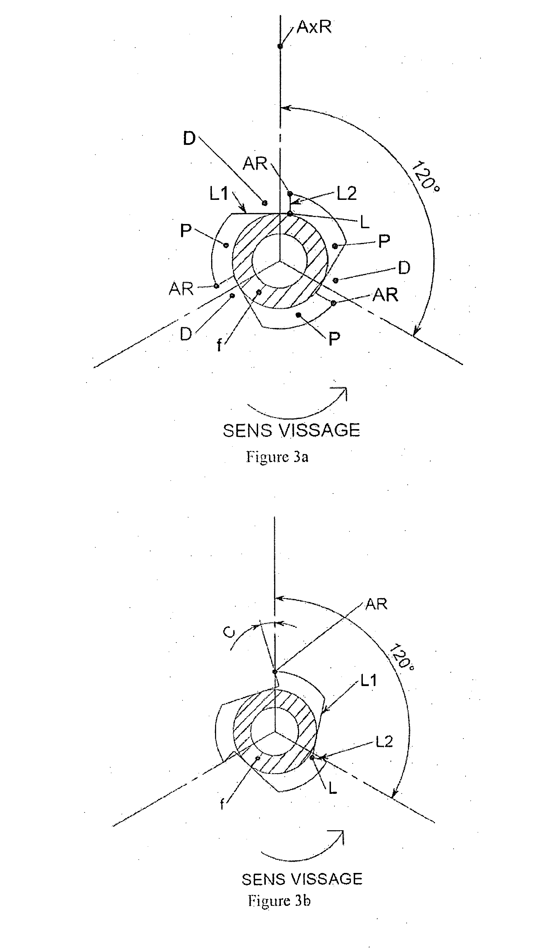

La figure 3a montre quant à elle une coupe transversale de la vis dans la zone A1a, c'est-à-dire au niveau des dents D. La coupe est identique dans la zone A2a et n'est donc pas représentée. La zone hachurée f correspond au fût canulé de la vis. Les dents D sont représentées au nombre de trois, et réalisées par enlèvement de matière correspondant au ½ rectangle (L1-L2). Les arêtes coupantes AR correspondent à l'intersection du segment L2 avec l'extérieur du filet P. (AxR) est un axe radial de la vis.

La figure 3b montre la même coupe après une rotation faible d'un angle (C). Cet angle (C) correspond à l'angle entre l'axe radial AxR, passant par l'arête coupante AR et le segment L2.

Afin d'obtenir une pénétration correcte, l'extrémité distale du fût, à savoir f1 en distal et f2 en proximal (correspondant au fonds de filet), est légèrement conique, tant en distal (A1a) qu'en proximal (A2a), d'un angle a1 en distal, et respectivement a2 en proximal.

Cette conicité du fût f est associée à une conicité de la crête du filet (angle (b1) en distal, respectivement (b2) en proximal) suffisamment importante pour permettre un engrenage immédiat de la vis dès l'initialisation de la perforation.

En effet le filet de la vis est taillé au plus bas de l'extrémité (A1a) en distal, respectivement (A2a) en proximal sur cette trajectoire conique, c'est-à-dire que la profondeur de filet augmente très rapidement ce qui permet d'avoir une hauteur de filet significative au plus près de l'extrémité.

Selon le mode de réalisation de l'invention plus particulièrement décrit ici, d'une part la somme des angles a1+b1 en distal, a2+b2 en proximal, définis entre, d'une part, l'axe principal de la vis (AP) et la conicité extérieure du fût f1 en distal et f2 en proximal, a1 en distal, a2 en proximal, et, d'autre part, entre l'axe principal de la vis (AP) et la ligne de crête des filets (P) du pas de vis, à savoir b1 en distal, b2 en proximal, est supérieur à 45° et d'autre part la partie d'attaque (la plus distale) de chaque filet présente une pluralité d'arêtes coupantes (AR) obtenues par enlèvement de matière.

Par ailleurs et comme on l'a vu, dans des modes de réalisation avantageux, les arêtes coupantes (AR) sont au nombre de trois, la somme des angles (a1+b1 en distal, resp.a2+b2 en proximal) est comprise entre 50° et 70°, et/ou l'angle (a1) en distal est compris entre 4° et 10° et l'angle (a2) en proximal est compris entre 10° et 20°.

En pratique, et à titre d'exemple non limitatif, pour une vis de 2 mm de diamètre, le diamètre distal de l'extrémité distale est de 1,8 mm, pour permettre une bonne pénétration. L'angle a1 est de 6°, l'angle a2 est de 15°, tandis que b1 est fixé à 50° et b2 à 40° pour permettre l'ancrage immédiat. C'est-à-dire a1+b1=56° et a2+b2= 55°.

En coupe transversale, les arêtes coupantes sont obtenues par l'usinage d'un L fermé par le diamètre extérieur du filet P de la vis.

Ce L est constitué par un grand coté L1 et un petit coté L2 (figure 3).

Enfin, afin de ne pas fragiliser la vis, la position du point d'intersection des segments de droite L1 et L2 (point (L)), c'est-à-dire la profondeur de l'entaille, ne vient en aucun cas mordre dans le fût principal.

Dans le mode de réalisation avantageux, plus particulièrement décrit la profondeur des arêtes coupantes AR est inférieure ou égale à la profondeur du pas de vis.

Enfin sur la figure 3a, la position du plan défini en coupe transversale par la droite L2 dont l'intersection avec le filet représente l'arête coupante AR est décalé en arrière (ou en négatif) (dans le sens de vissage), par rapport à un axe radial de la vis (AxR) de telle sorte qu'en tournant comme on voit sur la figure 3b, ces arêtes AR attaquent l'os avec un angle C, défini en coupe transversale entre l'axe radial passant par AR et la droite L2.

De même on notera en référence à la figure 3a que les arêtes coupantes AR sont décalées en négatif par rapport à un axe radial à la vis, c'est-à-dire en arrière par rapport au sens de rotation de la vis au vissage, par exemple les arêtes coupantes (AR) sont décalées en arrière pour présenter un angle d'attaque de l'os au vissage C (en distal et en proximal) compris entre 5° et 12°.

En pratique à titre d'exemple pour une vis de diamètre nominal 2,5 mm, un décalage faible de 0,15 mm permet d'obtenir un angle (C) de 7°.

Afin d'améliorer l'évacuation de copeaux d'os, une dépouille de 10° en arrière dans le sens du vissage peut être réalisée sur chaque dent (D).

Egalement l'extrémité externe de la tête peu être entièrement conique, pour enfouissement dans l'os, ou présenter une tête plate pour prendre appui sur la surface externe de l'os.

On va maintenant décrire en référence à la figure 1 la mise en place d'une vis selon l'invention.

Le chirurgien va tout d'abord rapprocher les deux morceaux d'os en les juxtaposant dans leur position de solidification.

Puis, sans préparation initiale ou pré-taraudage, il va visser la vis au travers de la partie d'os dite supérieure.

La partie distale étant conique, la pénétration s'effectue parfaitement jusqu'à ce que la partie proximale reste en contact avec l'os.

Les pas différents des vis exercent alors un effet de rapprochement et de mise en compression, des parties d'os qui est augmenté en fin de vissage par l'aspect conique des extrémités des deux parties distales et proximales de façon accrue.

De façon étonnante la combinaison des deux vissages à effet de cône, telle que décrite ci-avant, augmente la stabilité de l'ensemble et autorise une consolidation exceptionnelle.

Comme il va de soi et comme il résulte de ce qui précède, la présente invention ne se limite pas aux modes de réalisation plus particulièrement décrits. Elle en embrasse au contraire toutes les variantes.

## Revendications

1. Vis d'ostéosynthèse et d'arthrodèse pour la chirurgie orthopédique notamment de la main et du pied, canulée ou non, présentant le long de son axe principal (AP) trois parties successives, à savoir
une partie distale comprenant des filets (A1),
une partie centrale ou fût central lisse (f)
et une partie proximale ou tête comprenant des filets (A2) et pour laquelle, la somme des angles (a2+b2) à savoir d'une part un angle (a2) défini entre l'axe principal de la vis (AP) et la conicité extérieure du fût (f2) et d'autre part un angle (b2) défini entre l'axe principal de la vis (AP) et la ligne de crête des filets (P) du pas de vis, est supérieure à 45°,
la partie distale ayant un diamètre externe légèrement inférieur à celui de la partie proximale et présentant un pas de vis légèrement supérieur à celui de la partie proximale, permettant une mise en compression des deux parties osseuses à souder, la zone d'attaque de l'os en partie proximale (A2a) étant conique auto-perforante,
**caractérisé en ce que** l'extrémité de la partie distale est conique,
**en ce que** la somme des angles (a1 + b1) de la partie distale à savoir d'une part un angle (a1) défini entre l'axe principal de la vis (AP) et la conicité extérieure de fût (f1) et d'autre part un angle (b1) défini entre l'axe principal de la vis (AP) et la ligne de crêtes des filets (P) du pas de vis, est supérieure à 45°,
et **en ce que** la partie d'attaque (la plus distale) de chaque filet présente une pluralité d'arêtes coupantes (AR) obtenues par enlèvement de matière.

2. Vis selon la revendication 1, **caractérisée en ce que** les arêtes coupantes (AR) sont décalées en négatif par rapport à un axe radial à la vis, c'est-à-dire en arrière par rapport au sens de rotation de la vis au vissage.

3. Vis selon la revendication 1, **caractérisée en ce que** les arêtes coupantes (AR) sont décalées en arrière pour présenter un angle d'attaque de l'os au vissage (C) en distal et en proximal) compris entre 5° et 12°.

4. Vis selon l'une quelconque des revendications 1 à 3, **caractérisée en ce que** les arêtes coupantes (AR) sont préférentiellement au nombre de trois.

5. Vis selon l'une quelconque des revendications 1 à 4, **caractérisée en ce que** la profondeur des arêtes coupantes (AR) est inférieure ou égale à la profondeur du pas de vis.

6. Vis selon la revendication 5, **caractérisée en ce que** la profondeur des arêtes coupantes (AR) est égale à la profondeur du pas de vis.

7. Vis selon l'une quelconque des revendications 1 à 6, **caractérisée en ce que** la somme des angles (a1+b1 en distal, a2+b2 en proximal) est comprise entre 50° et 70°.

8. Vis selon la revendication 7, **caractérisée en ce que** l'angle (a1) en distal est compris entre 4° et 10° et l'angle (a2) en proximal est compris entre 10° et 20°.

9. Vis selon l'une quelconque des revendications 1 à 8, **caractérisée en ce que** les arêtes coupantes (AR) présentent une dépouille de 10° environ en arrière dans le sens de vissage.

10. Vis selon l'une quelconque des revendications 1 à 9, **caractérisée en ce que** la vis présente une tête plate.

## Patentansprüche

1. Kanülierte oder nicht kanülierte Osteosynthese- und Arthrodese-Schraube für die orthopädische Chirurgie insbesondere der Hand und des Fußes mit drei aufeinanderfolgenden Bereichen entlang ihrer Hauptachse (AP), und zwar
- einen distalen Bereich mit Gewinden (A1),
- einen zentralen glatten Bereich bzw. Schaft (f) und
- einen proximalen Bereich bzw. Kopf mit Gewinden (A2), bei dem die Summe der Winkel (a2+b2), und zwar einerseits eines Winkels (a2) zwischen der Hauptachse der Schraube (AP) und der äußeren Kegelform des Schafts (f2) und andererseits eines Winkels (b2) zwischen der Hauptachse der Schraube (AP) und der Gratlinie der Gewindegänge (P) des Schraubenganges größer als 45° ist,
wobei der distale Bereich einen Außendurchmesser aufweist, der geringfügig kleiner ist als derjenige des proximalen Bereichs, und einen Schraubengang aufweist, der geringfügig größer ist als derjenigen des proximalen Bereichs, wodurch die zwei miteinander zu verbindenden Knochenteile zusammengedrückt werden, wobei der Knochenangriffsbereich in dem proximalen Bereich (A2a) kegelförmig und selbstschneidend ist,
**dadurch gekennzeichnet,**
**dass** das Ende des distalen Bereichs kegelförmig ist, dass die Summe der Winkel (a1+b1) des distalen Bereichs, und zwar einerseits eines Winkels (a1) zwischen der Hauptachse der Schraube (AP) und der äußeren Kegelform des Schafts (f1) und andererseits eines Winkels (b1) zwischen der Hauptachse der Schraube (AP) und der Gratlinie der Gewindegänge (P) des Schraubenganges größer als 45° ist,
und
**dass** der (distalste) Angriffsbereich jedes Gewindes eine Vielzahl von Schneidkanten (AR) aufweist, die durch Materialabtrag erzielt werden.

2. Schraube nach Anspruch 1,
**dadurch gekennzeichnet,**
**dass** die Schneidkanten (AR) negativ versetzt sind in Bezug auf eine zur Schraube radiale Achse, das heißt nach hinten in Bezug auf die Drehrichtung der Schraube beim Schrauben.

3. Schraube nach Anspruch 1,
**dadurch gekennzeichnet,**
**dass** die Schneidkanten (AR) nach hinten versetzt sind, so dass beim Schrauben (C) distal und proximal ein zwischen 5° und 12° liegender Knochenangriffswinkel entsteht.

4. Schraube nach einem beliebigen der Ansprüche 1 bis 3,
**dadurch gekennzeichnet,**
**dass** die Anzahl der Schneidkanten (AR) vorzugsweise drei beträgt.

5. Schraube nach einem beliebigen der Ansprüche 1 bis 4,
**dadurch gekennzeichnet,**
**dass** die Tiefe der Schneidkanten (AR) geringer oder gleich der Tiefe des Schraubenganges ist.

6. Schraube nach Anspruch 5,
**dadurch gekennzeichnet,**
**dass** die Tiefe der Schneidkanten (AR) gleich der Tiefe des Schraubenganges ist.

7. Schraube nach einem beliebigen der Ansprüche 1 bis 6,
**dadurch gekennzeichnet,**
**dass** die Summe der Winkel (a1+b1 im distalen Bereich, a2+b2 im proximalen Bereich) zwischen 50° und 70° liegt.

8. Schraube nach Anspruch 7,
**dadurch gekennzeichnet,**
**dass** der Winkel (a1) im distalen Bereich zwischen 4° und 10° liegt und der Winkel (a2) im proximalen Bereich zwischen 10° und 20° liegt.

9. Schraube nach einem beliebigen der Ansprüche 1 bis 8,
**dadurch gekennzeichnet,**
**dass** die Schneidkanten (AR) eine Abschrägung von ca. 10° nach hinten in der Schraubrichtung aufweisen.

10. Schraube nach einem beliebigen der Ansprüche 1 bis 9,
**dadurch gekennzeichnet,**
**dass** die Schraube einen Flachkopf aufweist.

## Claims

1. Screw for osteosynthesis and arthrodesis for orthopaedic surgery notably of the hand and the foot, cannulated or not, having three successive parts along its principal axis (AP), namely
a distal part comprising threads (A1),
a central part or central shank which is smooth (f) and
a proximal part or head comprising threads (A2) and for which the sum of the angles (a2 + b2), namely on the one hand an angle (a2) defined between the principal axis of the screw (AP) and the external taper of the shank (f2) and on the other hand an angle (b2) defined between the principal axis of the screw (AP) and the crest line of the threads (P) of the pitch of the screw, is greater than 45°,
the distal part having an external diameter which is slightly less than that of the proximal part and exhibiting a screw pitch which is slightly greater than that of the proximal part, allowing the two bone parts to be fused to be placed under compression, the zone of attack on the bone in the proximal part (A2a) being conical and self-perforating,
**characterised in that** the end of the distal part is conical,
**in that** the sum of the angles (a1 + b1) of the distal part, namely on the one hand an angle (a1) defined between the principal axis of the screw (AP) and the external taper of the shank (f1) and on the other hand an angle (b1) defined between the principal axis of the screw (AP) and the crest line of the threads (P) of the pitch of the screw, is greater than 45°,
and **in that** the attacking (most distal) part of each thread has a plurality of cutting edges (AR) obtained by removal of material.

2. Screw according to claim 1, **characterised in that** the cutting edges (AR) are negatively offset with respect to an axis radial to the screw, i.e. to the back with respect to the direction of rotation of the screw when screwing in.

3. Screw according to claim 1, **characterised in that** the cutting edges (AR) are offset to the back to present an angle of attack on the bone when screwing in (C) distally and proximally, of between 5° and 12°.

4. Screw according to any one of claims 1 to 3, **characterised in that** the cutting edges (AR) are preferably three in number.

5. Screw according to any one of claims 1 to 4, **characterised in that** the depth of the cutting edges (AR) is less than or equal to the depth of the pitch of the screw.

6. Screw according to claim 5, **characterised in that** the depth of the cutting edges (AR) is equal to the depth of the pitch of the screw.

7. Screw according to any one of claims 1 to 6, **characterised in that** the sum of the angles (a1 + b1 distally, a2 + b2 proximally) is between 50° and 70°.

8. Screw according to claim 7, **characterised in that** the angle (a1) distally is between 4° and 10° and the angle (a2) proximally is between 10° and 20°.

9. Screw according to any one of claims 1 to 8, **characterised in that** the cutting edges (AR) have a taper of approximately 10° to the back in the screwing direction.

10. Screw according to any one of claims 1 to 9, **characterised in that** the screw has a flat head.
